# EUROPEAN PATENT SPECIFICATION

(11) **EP 1 787 647 B1**
(45) Date of publication and mention of the grant of the patent: **20.06.2012**
(21) Application number: 05777027.3
(22) Date of filing: 02.09.2005
(51) Int. Cl.: A61K 31/4415, A61K 31/41, A61K 31/4178, A61K 31/4184, A61K 31/4245, A61K 45/06, A61P 13/12, A61K 31/4166

(54) **REMEDY FOR GLOMERULAR DISEASES**
MITTEL ZUR BEHANDLUNG VON GLOMERULÄRER ERKRANKUNGEN
MEDICAMENT CONTRE LES MALADIES GLOMERULAIRES

(30) Priority: 06.09.2004 JP 2004258027
(43) Date of publication of application: 23.05.2007
(73) Proprietor: Kowa Company, Ltd., Nagoya-shi, Aichi 460-8625 (JP)
(72) Inventor: NAKAGAWA, Takashi, Hachioji-shi, Tokyo 1930801 (JP)
(74) Representative: Hartz, Nikolai
(86) International application number: PCT/JP2005/016095
(87) International publication number: WO 2006/028007

(56) References cited:
- WO-A1-02/083127
- WO-A1-02/092081
- WO-A1-2004/112788
- JP-A- 11 512 432
- JP-A- 2003 507 418
- WILLIAMS MARK E ET AL: "A phase 2 clinical trial of pyridoxamine (PyridorinTM) in type 1 and type 2 diabetic patients with overt nephropathy (PYR-206)." JOURNAL OF THE AMERICAN SOCIETY OF NEPHROLOGY, vol. 14, no. Abstracts Issue, November 2003 (2003-11), page 7A, XP008085714 & MEETING OF THE AMERICAN SOCIETY OF NEPHROLOGY RENAL WEEK; SAN DIEGO, CA, USA; NOVEMBER 12-17, 2003 ISSN: 1046-6673
- BELL DAVID S H ET AL: "Investigation of the safety and efficacy of pyridoxamine (Pyridorin (TM)) in patients with diabetic nephropathy (PYR-206)" DIABETES, vol. 53, no. Suppl. 2, June 2004 (2004-06), page A119, XP008085718 & 64TH ANNUAL MEETING OF THE AMERICAN-DIABETES-ASSOCIATION; ORLANDO, FL, USA; JUNE 04 -08, 2004 ISSN: 0012-1797
- MCGILL JANET B ET AL: "A phase 2 clinical investigation of pyridoxamine (Pyridorin (TM)) in type 1 and type 2 diabetic patients with overt diabetic nephropathy (PYR-205/207)" DIABETES, vol. 53, no. Suppl. 2, June 2004 (2004-06), page A138, XP008085737 & 64TH ANNUAL MEETING OF THE AMERICAN-DIABETES-ASSOCIATION; ORLANDO, FL, USA; JUNE 04 -08, 2004 ISSN: 0012-1797
- DISTILLER L A ET AL: "Clinical investigation of pyridoxamine in Type 1 and Type 2 diabetic patients with overt diabetic nephropathy (PYR-205/207)" DIABETOLOGIA, vol. 47, no. Suppl. 1, August 2004 (2004-08), page A89, XP008085719 & 40TH ANNUAL MEETING OF THE EUROPEAN-ASSOCIATION-FOR-THE-STUDY-OF-DIAB ETES; MUNICH, GERMANY; SEPTEMBER 05 -09, 2004 ISSN: 0012-186X
- DEGENHARDT THORSTEN P ET AL: "Pyridoxamine inhibits early renal disease and dyslipidemia in the streptozotocin-diabetic rat" KIDNEY INTERNATIONAL, vol. 61, no. 3, March 2002 (2002-03), pages 939-950, XP002457970 ISSN: 0085-2538
- MIYATA T. ET AL: 'Angiotensin II receptor antagonists and angiotensin-converting enzyme inhibitors lower in vitro the formation of advanced glycation end products: biochemical mechanisms' J.AM.SOC.NEPHOL. vol. 13, no. 10, 2002, pages 2478 - 2487, XP002998918
- ISCHIKAWA N.: 'Tonyobyosei Kansho ni Taisuru Aratana Hogo Sayoyaku no Tansaku Carbonyl Stress Sogaizai. (Carbonyl stress inhibitor towards new therapeutic interventions in diabetic nephropathy)' JAPANSE JOURNAL OF CLINICAL MEDICINE vol. 62, no. 5, 28 May 2004, pages 389 - 392, XP002998919

## Description

### Technical Field

The present invention relates to a drug useful for the prevention or therapy of glomerular diseases, in particular, progressive chronic glomerular diseases.

### Background Art

In Japan, the number of diabetic patients, patients with suspected diabetes, and people who are likely to develop diabetes is currently said to exceed 20 million. Among complications caused by diabetes, diabetic nephropathy has been increasing in incidence year after year, and the incidence of diabetic nephropathy has already exceeded that of chronic glomerular nephritis, making diabetic nephropathy the most common complication of diabetes.
The biggest problem associated with development of diabetic nephropathy is very high percent progression thereof to end-stage renal failure (including renal dialysis).
Similar to the case of diabetic nephropathy, progression of chronic glomerular nephritis to renal dialysis causes serious social problems, including an increase in medical costs. Therefore, keen demand has arisen for a drug which suppresses or prevents progression of diabetic nephropathy or chronic glomerular nephritis.
In the case of such a progressive renal disease, proliferation of mesangial cells and hyperplasia of mesangial matrix are observed in glomeruli. Particularly, such a lesion is considered to greatly contribute to progression of the disease. In many cases, such a lesion also extends to renal tubules or interstitial regions in the vicinity of glomeruli.

In recent years, advanced glycation end products (AGEs) have become of interest as a factor for causing diabetic nephropathy (Non-Patent Document 1). AGEs are considered to be generally produced through reaction between sugars and proteins; i.e., Maillard reaction.

Clinical studies have shown that a large amount of AGEs are detected in the plasma of renal dialysis patients with diseases not caused by diabetes, and have reported a number of new AGE production systems which are not derived from hyperglycemia (Non-Patent Document 2).

AGEs have been known to adversely affect many renal functions, including renal circulation kinetics, as well as filtration mechanism of basement membrane. Also, AGEs have been known to act on numerous AGE-related receptors (e.g., receptor for AGE: RAGE) which are present in renal constituent cells such as mesangial cells, thereby producing lesion factors (e.g., cytokines and growth factors).
Therefore, suppression of AGE production has been considered to lead to suppression of progression of a progressive renal disease, and in view of the above, drugs aiming at suppressing AGE production have been developed. Among such drugs, pyridoxamine (4-(aminomethyl)-5-hydroxy-6-methyl-3-pyridinemethanol), which is a vitamin B6 derivative, and a salt thereof have been reported to be effective against diabetic nephropathy since it inhibits an AGE formation process, thereby suppressing AGE production (Patent Document 1).

Meanwhile, angiotensin II has been known to promote progression of renal diseases. Specifically, angiotensin II contracts efferent arterioles of renal glomeruli, to thereby increase the internal pressure of the glomeruli, and to promote leakage of a large amount of plasma protein into urine.
Currently, a number of angiotensin II-related drugs are used for the purpose of suppressing progression of renal disorders, including an angiotensin-converting enzyme inhibitor, which suppresses production of angiotensin II, and an angiotensin II receptor antagonist, which inhibits the effect of angiotensin II on angiotensin type 1 receptor (AT1) (Patent Document 2).
In recent years, an angiotensin II receptor antagonist has been reported to have the effect of inhibiting AGE production (Patent Document 3).

However, single administration of pyridoxamine or an angiotensin II receptor antagonist exhibits insufficient effects on progressive renal diseases. Therefore, demand has arisen for development of a new drug exhibiting further excellent effects.
Patent Document 1: WO 97/09981 pamphlet
Patent Document 2: WO 02/092081 pamphlet
Patent Document 3: WO 02/083127 pamphlet
Non-Patent Document 1: Am. J. Pathol., 164 (4): 1389-97, 2004
Non-Patent Document 2: Diahetes Res. Clin. Pract., 61 (3): 145-153, 2003; Nephrol. Dial. Transplant., 18 (9): 1716-25, 2003
Williams et al., Journal of the American Society of Nephrology, 14, abstracts issue, November 2003, page 7A describes results of phase 2 clinical trial PYR-206 of pyridoxamine in type I and type 2 diabetic patients with overt nephropathy. It is not disclosed that an agent comprising pyridoxamine or a salt thereof and an angiotensin II receptor antagonist can be used in the therapy or prevention of a glomerular disease, in which the glomerular disease is IgA nephropathy, focal glomerulosclerosis or membranous nephropathy. It is also not disclosed that an agent comprising pyridoxamine or a salt thereof and candesartan cilexetil or a salt thereof can be used in the therapy or prevention of a glomerular disease. Bell et al., Diabetes 53, no. Suppl. 2, June 2004, page A119 also relates to clinical study PYR-206.
McGill et al., Diabetes 53, no. Suppl. 2, June 2004, page A138 describes phase 2 clinical study PYR-205/207 wherein pyridoxamine was tested in type 1 and type 2 diabetic patients with overt diabetic nephropathy. It is not disclosed that an agent comprising pyridoxamine or a salt thereof and an angiotensin II receptor antagonist can be used in the therapy or prevention of a glomerular disease in which the glomerular disease is IgA nephropathy, focal glomerulosclerosis or membranous nephropathy. It is also not disclosed that an agent comprising pyridoxamine or a salt thereof and candesartan cilexetil or a salt thereof can be used in the therapy or prevention of a glomerular disease. Distiller et al., Diabetologia 47, no. Suppl. 1, August 2004, page A89 also describes clinical study PYR-205/207.
Miyata et al., J. Am. Soc. Nephrol. 13 (2002) 2478-2487 reports that angiotensin II receptor antagonists and angiotensin-converting enzyme inhibitors lower *in vitro* the formation of advanced glycation end products. Miyata et al. does not relate to primary glomerular diseases.

### Disclosure of the Invention

### Problems to be Solved by the Invention

An object of the present invention is to provide a preventive or therapeutic agent for a glomerular disease, in particular, a progressive chronic glomerular disease. As used herein, the expression "prevention or therapy of a glomerular disease" encompasses suppression of progression of the disease, as well as amelioration of the disease.

### Means for Solving the Problems

In view of the foregoing, the present inventors have conducted extensive studies, and as a result have found that when pyridoxamine, which is an AGE production inhibitor, and an angiotensin II receptor antagonist are used in combination, progression of renal diseases is considerably suppressed, as compared with the case where these drugs are used singly, and therefore use of these drugs in combination is useful for the prevention or therapy of progressive chronic glomerular diseases. The present invention has been accomplished on the basis of this finding.

Accordingly, the present invention provides a preventive or therapeutic agent for a glomerular disease containing pyridoxamine or a salt thereof, and an angiotensin II receptor antagonist, as defined in any one of claims 1 to 3. Further, a method for preventing or treating a glomerular disease comprising administering, in effective amounts, pyridoxamine or a salt thereof, and an angiotensin II receptor antagonist to a subject in need thereof is described.
The present invention also provides use of pyridoxamine or a salt thereof, and an angiotensin II receptor antagonist for producing a preventive or therapeutic agent for a glomerular disease as defined in any one of claims 4 to 6.

### Effects of the Invention

The preventive or therapeutic agent for glomerular diseases of the present invention is useful for the prevention or therapy of glomerular diseases (in particular, progressive chronic glomerular diseases), including IgA nephropathy, focal glomerulosclerosis, membranous nephropathy, membranous proliferative nephritis, and diabetic nephropathy.

### Brief Description of the Drawings

[Fig. 1] Fig. 1 shows change in survival rate (%) of 5/6 nephrectomized rats.
[Fig. 2] Fig. 2 shows time-course change in urinary total protein excretion of 5/6 nephrectomized rats.
[Fig. 3] Fig. 3 shows time-course change in plasma creatinine level of 5/6 nephrectomized rats.
[Fig. 4] Fig. 4 shows systolic blood pressure data of 5/6 nephrectomized rats.

### Best Mode for Carrying Out the Invention

As used herein, "pyridoxamine (4-(aminomethyl)-5-hydroxy-6-methyl-3-pyridinemethanol)," which is a vitamin B6 derivative exhibiting the effect of inhibiting AGE production, encompasses a salt thereof. Examples of the pyridoxamine salt include acid addition salts, such as a hydrochloride, a nitrate, a sulfate, a phosphate, an oxalate, a maleate, a succinate, and a methanesulfonate. Among them, hydrochlorides are preferred, with dihydrochloride being particularly preferred.

An angiotensin II receptor antagonist, which is employed in the present invention, exhibits, for example, blood pressure lowering effect or heart failure ameliorating effect by antagonizing angiotensin II receptor. Examples of the angiotensin II receptor antagonist employed include losartan (2-butyl-4-chloro-1-[2'-(1H-tetrazol-5-yl)biphenyl-4-ylmethyl])-1H-imidazole-5-methanol: JP-A-1988-23868, US Patent No. 5138069), candesartan cilexetil 1-(cyclohexyloxycarbonyloxy)ethyl 2-ethoxy-1-[2'-(1H-tetrazol-5-yl)biphenyl-4-ylmethyl]-1H-benzimidazole-7-carboxylate: JP-A-1992-364171, US Patent No. 5196444), valsartan ((S)-N-valeryl-N-[2'-(1H-tetrazol-5-yl)biphenyl-4-ylmethyl]valine: JP-A-1992-235149, US Patent No. 5399578), telmisartan (4'-[[2-n-propyl-4-methyl-6-(1-methylbenzimidazol-2-yl)benzimidazol-1-yl]methyl]biphenyl-2-carboxylic acid: JP-A-1992-346978, US Patent No. 5591762), irbesartan (2-N-butyl-4-spirocyclopentane-1-[2'-(1H-tetrazol-5-yl)biphenyl-4-ylmethyl]-2-imidazolin-5-one: Japanese kohyo Patent Publication No. 1992-506222, US Patent No. 5270317), olmesartan (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate: JP-A-1993-78328, US Patent No. 5616599), eprosartan ((E)-3-[2-butyl-1-[(4-carboxyphenyl)methyl]imidazol-5-yl]-2-(2-thienylmethyl)-2-propenoic acid: European Patent Publication No. 403159, US Patent No. 5185351), and 2-ethoxy-1-[[2'-(4,5-dihydro-5-oxo-1,2,4-oxadiazol-3-yl)biphenyl-4-yl]methyl]benzimidazole-7-carboxylic acid (JP-A-1993-271228, European Patent Publication No. 520423). As used herein, "angiotensin II receptor antagonist" encompasses a pharmacologically acceptable salt thereof. Examples of such a salt include salts with inorganic acids (e.g., hydrochloric acid, sulfuric acid, and nitric acid); salts with organic acids (e.g., citric acid, ascorbic acid, lactic acid, p-toluenesulfonic acid, methanesulfonic acid, and fumaric acid); alkali metal salts (e.g., a sodium salt and a potassium salt); salts with alkaline earth metals (e.g., calcium and magnesium); and ammonium salts.

The angiotensin II receptor antagonist employed is preferably losartan potassium, candesartan cilexetil or a salt thereof, valsartan, telmisartan, irbesartan, olmesartan, eprosartan methanesulfonate, or 2-ethoxy-1-[[2'-(4,5-dihydro-5-oxo-1,2,4-oxadiazol-3-yl)biphenyl-4-yl]methyl]benzimidazole-7-carboxylic acid, particularly preferably candesartan cilexetil or a salt thereof. When candesartan cilexetil is employed in the form of salt, the salt form is preferably a hydrochloride, a methanesulfonate, or a potassium salt.

As used herein, "pyridoxamine" or "angiotensin II receptor antagonist or a salt thereof" also encompasses a hydrate thereof, as well as a solvate thereof with a pharmaceutically acceptable solvent.

The preventive or therapeutic agent for glomerular diseases of the present invention can be transformed, in accordance with directions for use, into pharmaceutical products of various forms. Examples of the product forms include powder, granules, fine granules, dry syrup, tablets, capsules, and injection.

Such pharmaceutical products may be produced, in consideration of its product form, through a routine method by appropriately mixing with, diluting with, or dissolving in a pharmaceutically employable additive, such as an excipient, a disintegrant, a binder, a lubricant, a diluent, a buffer, an isotonizing agent, a preservative, a humectant, an emulsifier, a dispersant, a stabilizer, or a solution adjuvant.

For example, a powder product may be prepared by admixing the essential ingredients with an appropriate optional additive such as an excipient or a lubricant. A tablet product may be prepared by mixing the essential ingredients with an appropriate optional additive such as an excipient, a disintegrant, a.binder, or a lubricant, followed by tableting of the resultant mixture through a routine method. If necessary, the thus-obtained tablets may be subjected to coating, to thereby yield film-coated tablets, sugar-coated tablets, etc.

An injection product may be in the form of a liquid formulation (aseptic aqueous or non-aqueous solution), an emulsion, or a suspension. Examples of non-aqueous carriers, diluents, solvents, and vehicles employed for preparing such an injection product include propylene glycol, polyethylene glycol, vegetable oils such as olive oil, and injectable organic acid esters such as ethyl oleate. The pharmaceutical composition may appropriately contain an adjuvant such as a preservative, a humectant, an emulsifier, or a dispersant.

The dose of the preventive or therapeutic agent for glomerular diseases of the present invention is appropriately determined in consideration of the body weight, age, or sex of a patient in need thereof, the progress of the disease of the patient, or other factors in relation to the patient. In the case of oral administration, the daily dose of pyridoxamine for an adult is 50 to 2,000 mg, whereas the daily dose of an angiotensin II receptor antagonist for an adult, which varies with the type of the antagonist, is generally 1 to 300 mg; for example, the daily dose of candesartan cilexetil is 1 to 50 mg. No particular limitation is imposed on the mixing ratio between pyridoxamine and an angiotensin II receptor antagonist, so long as the doses of these drugs fall within the aforementioned ranges. Preferably, oral administration is carried out once a day, or in a divided manner (several times a day).

As described below in an Example, in the test employing 5/6 nephrectomized rats of progressive renal failure model (plasma creatinine level at the start of drug administration: 1.5 mg/dL), in which drug administration was initiated at week five after surgery, administration of pyridoxamine and an angiotensin II receptor antagonist in combination according to the present invention exhibited excellent effects, as compared with the case of single administration of each drug. Specifically, administration of these drugs in combination (hereinafter may be referred to as "combined administration") exhibited potent effects on i) suppression of progression to renal failure (improvement of survival rate), ii) reduction in urinary total protein excretion, iii) suppression of impairment of renal function, iv) suppression of an increase in blood pressure, and v) suppression of glomerular tissue sclerosis, as compared with the case of single administration of these drugs. Particularly, the combined administration exhibited remarkable effect on i) suppression of progression to renal failure; i.e., the survival rate of rats in the combined administration group was maintained at 100% even 15 weeks after initiation of drug administration (i.e., 20 weeks after surgery).

Thus, administration of pyridoxamine and an angiotensin II receptor antagonist in combination prevents progression of progressive renal diseases to renal failure; i.e., the combined administration retards progression to renal dialysis.
The aforementioned combined administration according to the present invention is useful for the prevention or therapy for progressive chronic glomerular diseases, including IgA nephropathy, focal glomerulosclerosis, membranous nephropathy, membranous proliferative nephritis, type I diabetic nephropathy, and type II diabetic nephropathy.

### Example

The present invention will next be described in more detail by way of an Example.

### Example 1. Effect on 5/6 nephrectomized rat

Fifty-one male SD rats (seven weeks old, purchased from Japan SLC, Inc.) were anesthetized with pentobarbital, and the left kidney of each rat was exposed. Under a stereoscopic microscope, two of the left renal artery branches (generally, one renal artery is separated into three branches) were ligated and one central branch was left intact. When a renal artery branch is ligated, a portion controlled by the branch falls into ischemia, and such an ischemic renal portion can be visually detected. One week later, the right kidney was removed. That is to say, 5/6 of the kidneys was removed. Four weeks after surgery, blood was collected from each rat under unanesthetized conditions, and plasma creatinine level (Pcr) was measured. Pcr is a blood parameter indicative of renal function, and an increase in Pcr indicates impairment of renal function. After Pcr measurement, rats with considerably impaired renal function and rats with no impairment of renal function were eliminated, to thereby select 36 rats employed for the test.

Subsequently, on the basis of the thus-obtained Pcr data, these rats were divided into four groups in such a manner that the value of Pcr was averaged out among each group: control group (i.e., no drug administration group) (N = 9); pyridoxamine dihydrochloride administration group (administered in the form of aqueous solution (1.5 g/L)) (N = 9); candesartan cilexetil administration group ((1 mg/kg) administered orally once a day) (N = 9) ; and combined administration group (pyridoxamine dihydrochloride administered in the form of aqueous solution (1.5 g/L), and candesartan cilexetil (1 mg/kg) administered orally once a day) (N = 9). In addition to these four groups, one normal rat group (non-surgical group) was provided.

Pyridoxamine dihydrochloride was dissolved in purified water, and the aqueous solution was available to the rats ad libitum. The daily dose of pyridoxamine dihydrochloride was about 100 mg/kg. Candesartan cilexetil was suspended in 0.5% methyl cellulose sodium solution upon use, and the suspension was repeatedly orally administered.

Drug administration was initiated at week five after surgery (Pcr was about 1.5 mg/dL at the start of drug administration; i.e., renal function had already been impaired).
After initiation of drug administration, the number of surviving rats was recorded every day. Pcr was measured at week two, week four, week seven, week 11, and week 15 after initiation of drug administration. Urinary total protein excretion was measured at week four, week seven, week 11, and week 15 after initiation of drug administration (each measurement was performed after 18-hour urine accumulation). In addition, at week 15 after initiation of drug administration, systolic blood pressure was measured under unanesthetized conditions by means of a noninvasive blood pressure measuring apparatus.

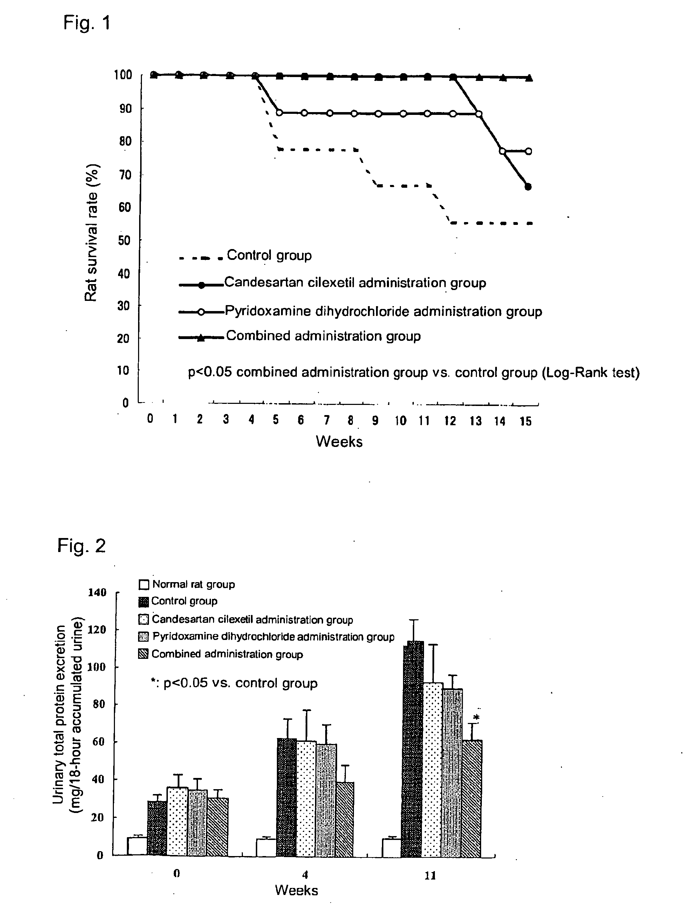

Fig. 1 shows the effect of single administration of pyridoxamine dihydrochloride, single administration of candesartan cilexetil, or administration of these drugs in combination on death due to renal failure caused by progressive impairment of renal function. As compared with the case of the control group (no drug administration group), in the pyridoxamine dihydrochloride administration group or candesartan cilexetil administration group, tendency for suppression of death due to renal failure was observed. In contrast, in the combined administration group, significant suppression of death due to renal failure was observed, and no death of rats was observed until at week 15 after initiation of drug administration, which was the same result as in the case of the normal rat group. The suppressive effect observed in the combined administration group was statistically significant with respect to the case of the control group. That is, the combined administration exhibits a potent effect in suppressing progression to renal failure due to progressive impairment of renal function.

Fig. 2 shows time-course change in urinary total protein excretion (UTP) of rats of the respective groups. In the combined administration group, tendency of reduction in UTP began to be observed at week four after initiation of drug administration, and UTP was maintained at a low level during subsequent drug administration period. Even at week 11 after initiation of drug administration, such a UTP reducing effect was statistically significant with respect to the case of the control group.

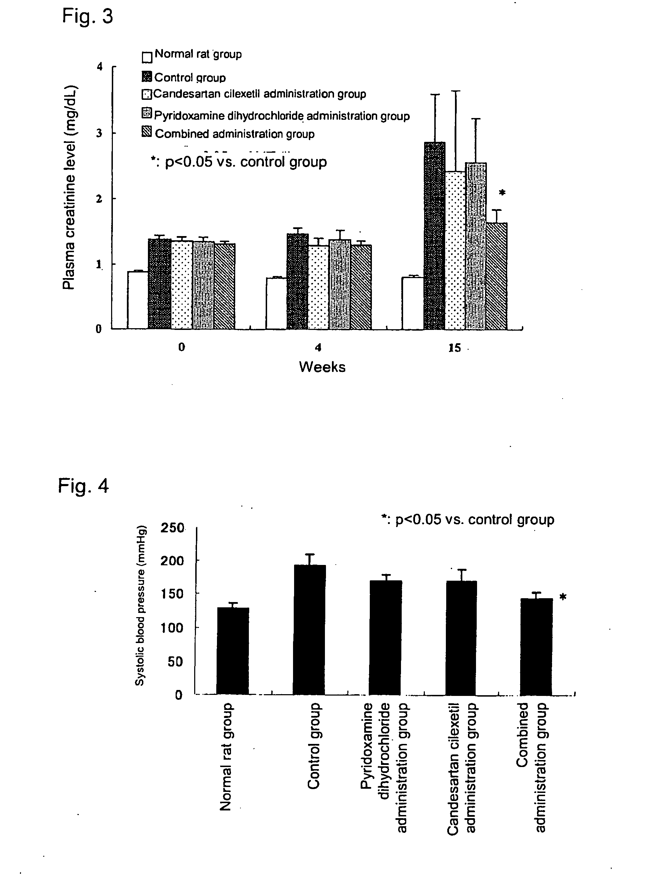

Fig. 3 shows time-course change in Pcr of rats of the respective groups. In the combined administration group, similar to the case of UTP, tendency of reduction in Pcr was began to be observed at week 4 after initiation of drug administration, and Pcr was maintained at a low level during subsequent drug administration period. At week 15 after initiation of drug administration, such a Pcr reducing effect was statistically significant with respect to the case of the control group.

Fig. 4 shows data of systolic blood pressure (SBP) of rats of the respective groups as measured at week 15 after initiation of drug administration. A significant systolic blood pressure reducing effect was observed only in the combined administration group.

At week 15 after initiation of drug administration, the renal tissue of each rat was observed, and the degree of glomerulosclerosis was evaluated on the basis of the below-described score. In the pyridoxamine dihydrochloride administration group, no reduction in score was observed, as compared with the case of the control group. In the candesartan cilexetil administration group, reduction in score was observed, and in the combined administration group, further reduction in score was observed. As used herein, the term "score" refers to the degree of glomerulosclerosis; i.e., the degree of hyperplasia of mesangial matrix in glomeruli. Fifty glomeruli per renal tissue piece were observed for evaluation. Score 0 was assigned to a glomerulus similar to that of rats in the normal rat group; score 1 was assigned to a glomerulus in which the area of mesangial matrix accounts for less than 25%; score 2 was assigned to a glomerulus in which the area of mesangial matrix accounts for 25% or more and less than 50%; score 3 was assigned to a glomerulus in which the area of mesangial matrix accounts for 50% or more and less than 75%; and score 4 was assigned to a glomerulus in which the area of mesangial matrix accounts for 75% or more. The average of the thus-obtained scores of the glomeruli was employed for evaluation of glomerulosclerosis.
The results are shown in Table 1.

**[Table 1]**

| | Glomerulosclerosis (score) |
|---|---|
| Normal rat group | 0.26 ± 0.14 |
| Control group | 1.81 ± 0.39 |
| Candesartan cilexetil administration group | 1.52 ± 0.45 |
| Pyridoxamine dihydrochloride administration group | 1.93 ± 0.35 |
| Combined administration group | 1.30 ± 0.3 |

## Claims

1. An agent comprising pyridoxamine or a salt thereof and an angiotensin II receptor antagonist for use in the therapy or prevention of a glomerular disease, wherein the glomerular disease is IgA nephropathy, focal glomerulosclerosis, membranous nephropathy or membranous proliferative nephritis.

2. The agent according to claim 1 for use according to claim 1, wherein the angiotensin II receptor antagonist is losartan potassium, candesartan cilexetil or a salt thereof, valsartan, telmisartan, irbesartan, olmesartan, eprosartan methanesulfonate, or 2-ethoxy-1-[[2'-(4,5-dihydro-5-oxo-1,2,4-oxadiazol-3-yl)biphenyl-4-yl]methyl]benzimidazole-7-carboxylic acid.

3. A preventive or therapeutic agent for use in the prevention or treatment of a glomerular disease, comprising pyridoxamine or a salt thereof and candesartan cilexetil or a salt thereof.

4. Use of pyridoxamine or a salt thereof, and an angiotensin II receptor antagonist for producing a preventive or therapeutic agent for the treatment or prevention of a glomerular disease, wherein the glomerular disease is IgA nephropathy, focal glomerulosclerosis, membranous nephropathy or membranous proliferative nephritis.

5. The use according to claim 4 for producing a preventive or therapeutic agent for the treatment or prevention of a glomerular disease as defined in claim 4, wherein the angiotensin II receptor antagonist is losartan potassium, candesartan cilexetil or a salt thereof, valsartan, telmisartan, irbesartan, olmesartan, eprosartan methanesulfonate, or 2-ethoxy-1-[[2'-(4,5-dihydro-5-oxo-1,2,4-oxadiazol-3-yl)biphenyl-4-yl]methyl]benzimidazole-7-carboxylic acid.

6. Use of pyridoxamine or a salt thereof and candesartan cilexetil or a salt thereof for producing a preventive or therapeutic agent for the treatment or prevention of a glomerular disease.

## Patentansprüche

1. Mittel umfassend Pyridoxamin oder ein Salz davon und einen Angiotensin-II-Rezeptorantagonisten zur Verwendung in der Therapie oder Prävention einer glomerulären Erkrankung, wobei die glomeruläre Erkrankung IgA-Nephropathie, fokale Glomerulosklerose, membranöse Nephropathie oder membranöse proliferative Nephritis ist.

2. Das Mittel gemäß Anspruch 1 zur Verwendung gemäß Anspruch 1, worin der Angiotensin-II-Rezeptorantagonist Losartan-Kalium, Candesartancilexetil oder ein Salz davon, Valsartan, Telmisartan, Irbesartan, Olmesartan, Eprosartan-Methansulfonat oder 2-Ethoxy-1-[[2'-(4,5-dihydro-5-oxo-1,2,4-oxadiazol-3-yl)biphenyl-4-yl]methyl]benzimidazol-7-carbonsäure ist.

3. Therapeutisches oder präventives Mittel zur Verwendung in der Therapie oder Prävention einer glomerulären Erkrankung, umfassend Pyridoxamin oder ein Salz davon und Candesartancilexetil oder ein Salz davon.

4. Verwendung von Pyridoxamin oder einem Salz davon und einem Angiotensin-II-Rezeptorantagonist zur Herstellung eines präventiven oder therapeutischen Mittels zur Behandlung oder Prävention einer glomerulären Erkrankung, worin die glomeruläre Erkrankung IgA-Nephropathie, fokale Glomerulosklerose, membranöse Nephropathie oder membranöse proliferative Nephritis ist.

5. Die Verwendung gemäß Anspruch 4, zur Herstellung eines präventiven oder therapeutischen Mittels für die Behandlung oder Prävention einer glomerulären Erkrankung wie in Anspruch 4 definiert, wobei der Angiotensin-II-Rezeptorantagonist Losartan-Kalium, Candesartancilexetil oder ein Salz davon, Valsartan, Telmisartan, Irbesartan, Olmesartan, Eprosartan-Methansulfonat oder 2-Ethoxy-1-[[2'-(4,5-dihydro-5-oxo-1,2,4-oxadiazol-3-yl)biphenyl-4-yl]methyl]benzimidazol-7-carbonsäure ist.

6. Verwendung von Pyridoxamin oder einem Salz davon und Candesartancilexetil oder einem Salz davon zur Herstellung eines präventiven oder therapeutischen Mittels zur Behandlung oder Prävention einer glomerulären Erkrankung.

## Revendications

1. Agent comprenant de la pyridoxamine ou un sel de celle-ci et un antagoniste de récepteur de l'angiotensine II, pour utilisation dans le traitement ou la prévention d'une maladie glomérulaire, dans lequel la maladie glomérulaire est une néphropathie à IgA, une glomérulosclérose focale, une glomérulonéphrite extra-membraneuse ou une néphrite proliférative extra-membraneuse.

2. Agent selon la revendication 1 pour utilisation selon la revendication 1, dans lequel l'antagoniste de récepteur de l'angiotensine II est le losartan potassique, le candésartan ciléxétil ou un sel de celui-ci, le valsartan, le telmisartan, l'irbésartan, l'olmésartan, le méthanesulfonate d'éprosartan, ou l'acide 2-éthoxy-1-[[2'-(4,5-dihydro-5-oxo-1,2,4-oxadiazol-3-yl)biphényl-4-yl]-méthyl]benzimidazole-7-carboxylique.

3. Agent prophylactique ou thérapeutique pour utilisation dans la prévention ou le traitement d'une maladie glomérulaire, comprenant de la pyridoxamine ou un sel de celle-ci et du candésartan ciléxétil ou un sel de celui-ci.

4. Utilisation de pyridoxamine ou d'un sel de celle-ci, et d'un antagoniste de récepteur de l'angiotensine II, pour produire un agent prophylactique ou thérapeutique destiné au traitement ou à la prévention d'une maladie glomérulaire, dans laquelle la maladie glomérulaire est une néphropathie à IgA, une glomérulosclérose focale, une glomérulonéphrite extra-membraneuse ou une néphrite proliférative extra-membraneuse.

5. Utilisation selon la revendication 4 pour produire un agent prophylactique ou thérapeutique destiné au traitement ou à la prévention d'une maladie glomérulaire, tel que défini dans la revendication 4, dans laquelle l'antagoniste de récepteur de l'angiotensine II est le losartan potassique, le candésartan ciléxétil ou un sel de celui-ci, le valsartan, le telmisartan, l'irbésartan, l'olmésartan, le méthanesulfonate d'éprosartan, ou l'acide 2-éthoxy-1-[[2'-(4,5-dihydro-5-oxo-1,2,4-oxadiazol-3-yl)biphényl-4-yl]méthyl]-benzimidazole-7-carboxylique.

6. Utilisation de pyridoxamine ou d'un sel de celle-ci et de candésartan ciléxétil ou d'un sel de celui-ci pour produire un agent prophylactique ou thérapeutique pour le traitement ou la prévention d'une maladie glomérulaire.
